# EUROPEAN PATENT SPECIFICATION

(11) **EP 2 649 954 B1**
(45) Date of publication and mention of the grant of the patent: **30.09.2015**
(21) Application number: 12828468.4
(22) Date of filing: 07.08.2012
(51) Int. Cl.: A61B 18/00, A61B 17/32

(54) **ULTRASONIC WAVE PROCESSING DEVICE**
VORRICHTUNG ZUR VERARBEITUNG VON ULTRASCHALLWELLEN
DISPOSITIF DE TRAITEMENT À ONDES ULTRASONORES

(30) Priority: 29.08.2011 US 201161528448 P
(43) Date of publication of application: 16.10.2013
(73) Proprietor: OLYMPUS MEDICAL SYSTEMS CORP., Tokyo 151-0072 (JP)
(72) Inventor: AKAGANE, Tsunetaka, Hachioji-shi, Tokyo 192-8512 (JP)
(74) Representative: Schicker, Silvia
(86) International application number: PCT/JP2012/070041
(87) International publication number: WO 2013/031490

(56) References cited:
- JP-A- H03 151 954
- JP-A- 2005 516 663
- JP-A- 2006 144 364
- US-A- 4 493 694
- US-A- 5 163 433
- US-A- 6 050 971
- US-A- 6 159 175
- US-A1- 2006 100 653

## Description

### Technical Field

The present invention relates to an ultrasonic treatment device (ultrasonic surgical device) configured to perform an ultrasonic treatment (ultrasonic surgery) including ultrasonic suction.

### Background Art

In general, there is used an ultrasonic treatment device configured to perform an ultrasonic treatment called ultrasonic suction. Such an ultrasonic treatment device includes a probe configured to transmit ultrasonic vibration from a proximal direction to a distal direction. The ultrasonic suction is performed by using a probe distal surface of the probe which ultrasonically vibrates, and is performed by utilizing a physical phenomenon called cavitation. Specifically, the probe repeats high speed vibration several ten thousand times per second by the ultrasonic vibration, and hence a pressure periodically fluctuates in a vicinity of the probe distal surface of the probe. When the pressure in the vicinity of the probe distal surface becomes lower than a saturated vapor pressure only for a very short time by the pressure fluctuation, micro-bubbles (cavities) are formed in a liquid of a body cavity or a liquid supplied (forwarded) from a liquid supplying unit to a vicinity of a position of a living tissue which is to be treated. Moreover, the formed bubbles disappear owing to a force which acts when the pressure in the vicinity of the probe distal surface increases (compresses). The above-mentioned physical phenomenon is called a cavitation phenomenon. By impact energy at the disappearance of the bubbles, a living tissue of, for example, hepatic cells which do not have elasticity is shattered (disintegrated) and emulsified. Moreover, in such an ultrasonic treatment device, a suction passage is provided along a longitudinal axis inside the probe. The shattered and emulsified living tissue passes through the suction passage from a suction port in a distal end portion of the probe, whereby suction and collection of the living tissue are accomplished. When the above operation is continued, the living tissue is resected. In this case, impact is absorbed in a living tissue such as a blood vessel having a high elasticity, and hence the living tissue having the high elasticity is not easily shattered (crushed), so that the living tissue is selectively shattered.

In Patent Literature 1, there is disclosed an ultrasonic treatment device which performs ultrasonic suction. In this ultrasonic treatment device, a suction passage is provided along a longitudinal axis in a probe. Moreover, a probe distal surface is provided with a distal suction port which communicates with the suction passage, and a distal end portion of a probe outer peripheral portion is provided with a suction port which communicates with the suction passage. A living tissue shattered and emulsified by cavitation is suctioned through the distal suction port or the suction port. Then, the living tissue passes through the suction passage, whereby suction and collection of the living tissue are accomplished. Furthermore, in this ultrasonic treatment device, a clearance portion is provided (interposed) between a sheath and the probe. A liquid supplied from a liquid supplying unit (liquid forwarding unit) is supplied through the clearance portion.

### Patent Literature 1: Jpn. Pat. Appln. KOKAI Publication No. 2001-29352

US 6,159,175 discloses a phacoemulsification instrument comprising a needle having an aspiration hole on a side of the needle. A sleeve has a single infusion hole on a circumferential surface thereof. A baffle may be provided on an exterior or an interior surface of the sleeve to direct fluid from the infusion hole away from the aspiration hole. The sleeve may have an accordion fold so as to be extendible in an axial direction of the needle.

US 6,050,971 discloses a phacoemulsification apparatus comprising an ultrasound transducer for vibrating a hollow needle, and a suction system. Fluid is infused to a treatment area through a channel formed between the needle and a handpiece wall. A first suction opening is located at an open end of the needle and a second suction opening is located proximally of the first suction opening on an outer peripheral surface of the needle. The handpiece has a sleeve extending around the needle and comprising an orifice at a distal surface thereof for infusing a fluid into the treatment area.

US 5,163,433 discloses an ultrasound treatment apparatus comprising an inner sheath and an outer sheath, wherein an area between the ultrasonic needle and the inner sheath sucks bodily tissue from a treatment area, and an area between the outer sheath and the inner sheath infuses fluid to the treatment area. The sheaths can move axially with respect to each other.

### Summary of Invention

### Technical Problem

In the ultrasonic treatment device of the above patent document 1, the whole distal suction port and the whole suction port are exposed to an outside. Therefore, the whole distal suction port and the whole suction port are easily closed with the living tissue (especially a membranous tissue). When the whole distal suction port and the whole suction port are closed, the suction passage has a negative pressure lower than an external pressure. When the suction passage is in the negative pressure state, the living tissue (the membranous tissue) firmly adheres to the probe in a vicinity of the distal suction port and in a vicinity of the suction port. As a result of the living tissue adhering to the probe, treatment performance in terms of ultrasonic suction deteriorates.

The present invention has been developed in view of the above problems, and an object thereof is to provide an ultrasonic treatment device which effectively prevents adhesion of a living tissue to a probe, and efficiently performs ultrasonic suction.

### Solution to Problem

To solve above mentioned problems, according to one aspect of the invention, an ultrasonic treatment device according to independent claim 1 is provided. The treatment device includes a sheath which includes a sheath outer peripheral portion and a sheath inner peripheral portion, and which is extended along a longitudinal axis; a probe which includes a probe distal surface and a probe outer peripheral portion, and which is inserted through the sheath in a state that the probe distal surface is positioned to a distal direction side of a distal end of the sheath; a suction passage defining portion which defines a suction passage along the longitudinal axis inside the probe; a clearance defining portion which defines a clearance between the sheath inner peripheral portion and the probe outer peripheral portion; a suction port defining portion which defines a suction port, the suction port communicating with the suction passage, in the probe outer peripheral portion in a state that a part of the suction port becomes a non-exposed portion whose outer peripheral direction side is covered with the sheath; and an opening defining portion which defines an opening in the sheath outer peripheral portion, the opening communicating with the clearance.

### Advantageous Effects of Invention

According to the present invention, an ultrasonic treatment device which effectively prevents adhesion of a living tissue to a probe and efficiently performs ultrasonic suction, can be provided.

### Brief description of Drawings

FIG. 1 is a schematic view showing an ultrasonic treatment device according to a first embodiment of the present invention;
FIG. 2 is a cross-sectional view schematically showing a constitution of a vibrator unit according to the first embodiment;
FIG. 3 is a perspective view schematically showing a probe according to the first embodiment;
FIG. 4 is a cross-sectional view schematically showing the probe according to the first embodiment;
FIG. 5 is a cross-sectional view taken along line V-V of FIG. 4;
FIG. 6 is a cross-sectional view schematically showing an inner constitution of a sheath and a coupling constitution of the sheath to a vibrator case according to the first embodiment;
FIG. 7 is a side view schematically showing a constitution of a distal end portion of the probe and a distal end portion of the sheath according to the first embodiment;
FIG. 8 is a cross-sectional view schematically showing the constitution of the distal end portion of the probe and the distal end portion of the sheath according to the first embodiment;
FIG. 9 is a cross-sectional view taken along line IX-IX of FIG. 8;
FIG. 10 is a perspective view schematically showing a constitution of a sheath inner peripheral portion in a vicinity of an opening of the sheath according to the first embodiment;
FIG. 11 is a schematic view showing the constitution of the sheath inner peripheral portion in the vicinity of the opening of the sheath according to the first embodiment;
FIG. 12 is a schematic view showing a constitution of a distal end portion of a probe and a distal end portion of a sheath according to a first comparative example not part of the invention ;
FIG. 13 is a schematic view showing a constitution of a distal end portion of a probe and a distal end portion of a sheath according to a second comparative example not part of the invention ;
FIG. 14 is a perspective view schematically showing a constitution of a sheath inner peripheral portion in a vicinity of an opening of a sheath according to a first modification of the first embodiment;
FIG. 15 is a schematic view showing the constitution of the sheath inner peripheral portion in the vicinity of the opening of the sheath according to the first modification of the first embodiment;
FIG. 16 is a perspective view schematically showing a constitution of a sheath inner peripheral portion in a vicinity of an opening of a sheath according to a second modification of the first embodiment;
FIG. 17 is a schematic view showing the constitution of the sheath inner peripheral portion in the vicinity of the opening of the sheath according to the second modification of the first embodiment;
FIG. 18 is a perspective view schematically showing a constitution of a sheath inner peripheral portion in a vicinity of an opening of a sheath according to a third modification of the first embodiment;
FIG. 19 is a schematic view showing the constitution of the sheath inner peripheral portion in the vicinity of the opening of the sheath according to the third modification of the first embodiment;
FIG. 20 is a side view schematically showing a constitution of a distal end portion of a probe and a distal end portion of a sheath according to a fourth modification of the first embodiment;
FIG. 21 is a cross-sectional view schematically showing the constitution of the distal end portion of the probe and the distal end portion of the sheath according to the fourth modification of the first embodiment;
FIG. 22 is a cross-sectional view taken along line 22-22 of FIG. 20;
FIG. 23 is a cross-sectional view taken along line 23-23 of FIG. 21;
FIG. 24 is a perspective view schematically showing a constitution of a distal end portion of a probe according to an arrangement not part of the present invention;
FIG. 25 is a cross-sectional view schematically showing the constitution of the distal end portion of the probe according to the arrangement of figure 24 and
FIG. 26 is a schematic view showing a constitution of the distal end portion of the probe and a distal end portion of a sheath according to the arrangement of figure 24.

### Description of Embodiments

### (First Embodiment)

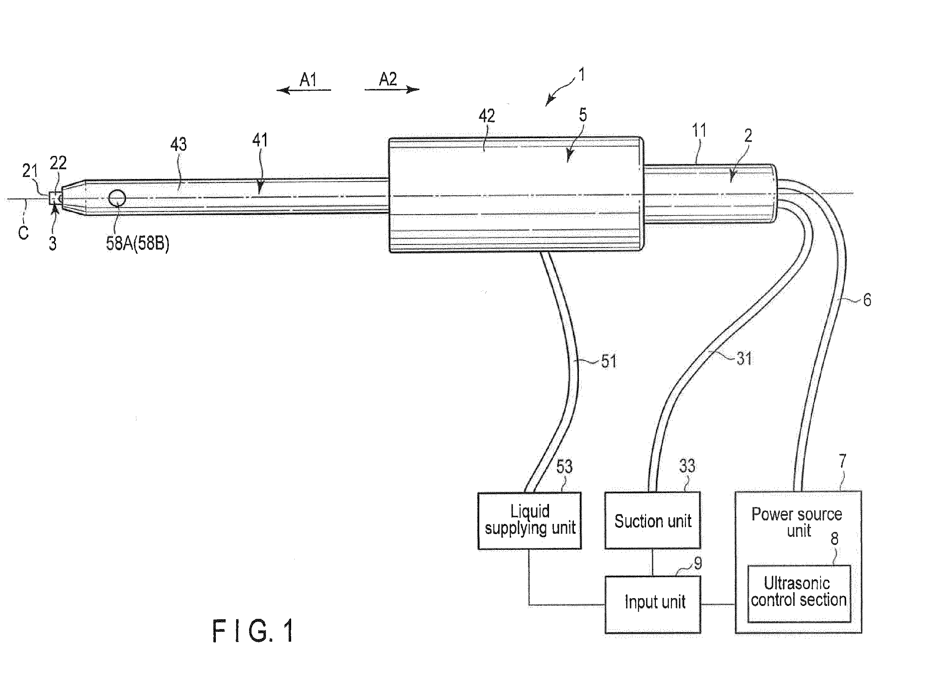

A first embodiment of the present invention will be described with reference to FIG. 1 to FIG. 13. FIG. 1 is a view showing an ultrasonic treatment device (ultrasonic surgical device) 1 of the present embodiment. It is to be noted that the ultrasonic treatment device 1 of the present embodiment is an ultrasonic suction device configured to selectively shatter (disintegrate) and emulsify a living tissue by utilizing cavitation caused by ultrasonic vibration, and configured to suction the shattered and emulsified living tissue. Moreover, the ultrasonic treatment device 1 has a longitudinal axis C. One of directions parallel to the longitudinal axis C is a distal direction (direction of arrow A1 in FIG. 1), and the other of the directions parallel to the longitudinal axis C is a proximal direction (direction of arrow A2 in FIG. 1).

As shown in FIG. 1, the ultrasonic treatment device (an ultrasonic treatment system) 1 includes an ultrasonic treatment instrument (ultrasonic treatment apparatus) including a vibrator unit (oscillator unit) 2, a probe 3 and a sheath unit 5; a power source unit 7; a suction unit 33; a water supplying unit 53; and an input unit 9.

The vibrator unit 2 includes a vibrator case (oscillator case) 11. One end of a cable 6 is connected to a proximal end portion of the vibrator case 11. The other end of the cable 6 is connected to the power source unit 7. The power source unit 7 includes an ultrasonic control section 8. The power source unit 7 is connected to the input unit 9, for example, a foot switch.

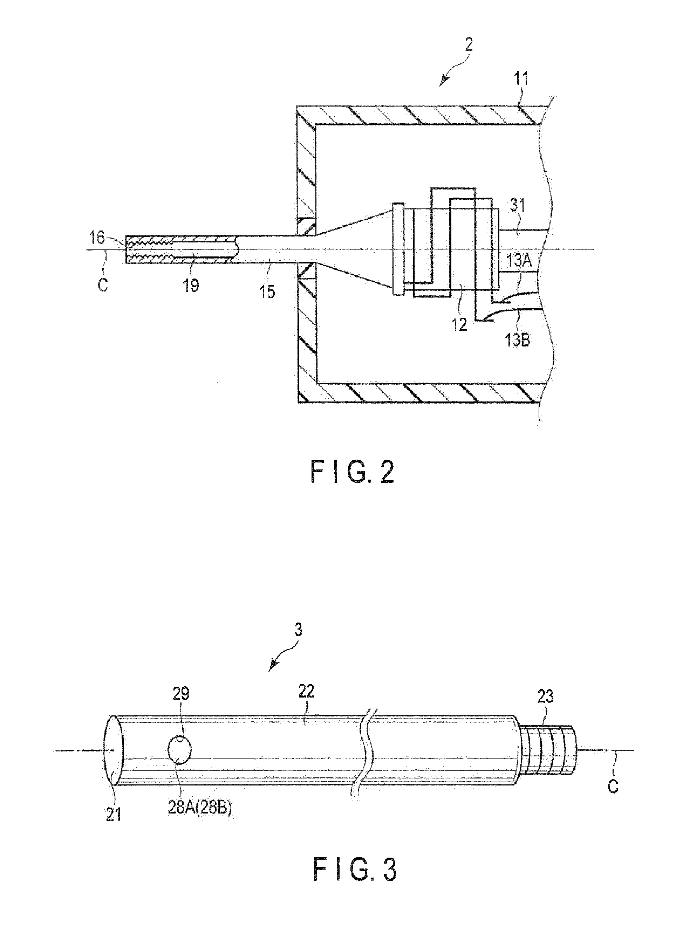

FIG. 2 is a view showing a constitution of the vibrator unit 2. As shown in FIG. 2, in the vibrator case 11, an ultrasonic vibrator (ultrasonic oscillator) 12 which includes a piezoelectric element configured to convert a current into the ultrasonic vibration is provided. One end of each of electrical signal lines 13A and 13B is connected to the ultrasonic vibrator 12. The electrical signal lines 13A and 13B pass through an inside of the cable 6, and the other ends of the electrical signal lines are connected to the ultrasonic control section 8 of the power source unit 7. When the current is supplied from the ultrasonic control section 8 to the ultrasonic vibrator 12 (the vibrator unit 2) through the electrical signal lines 13A and 13B, the ultrasonic vibration takes place in the ultrasonic vibrator 12. It is to be noted that the vibrator unit 2 and the power source unit 7 constitute an ultrasonic generation unit configured to generate the ultrasonic vibration. A horn 15 configured to enlarge an amplitude of the ultrasonic vibration is coupled to a distal direction side of the ultrasonic vibrator 12. The horn 15 is attached to the vibrator case 11. The ultrasonic vibrator 12 and the horn 15 are provided with a passage portion 19 about the longitudinal axis C. Moreover, in a distal end portion of an inner peripheral surface of the horn 15, an internal thread portion 16 is formed.

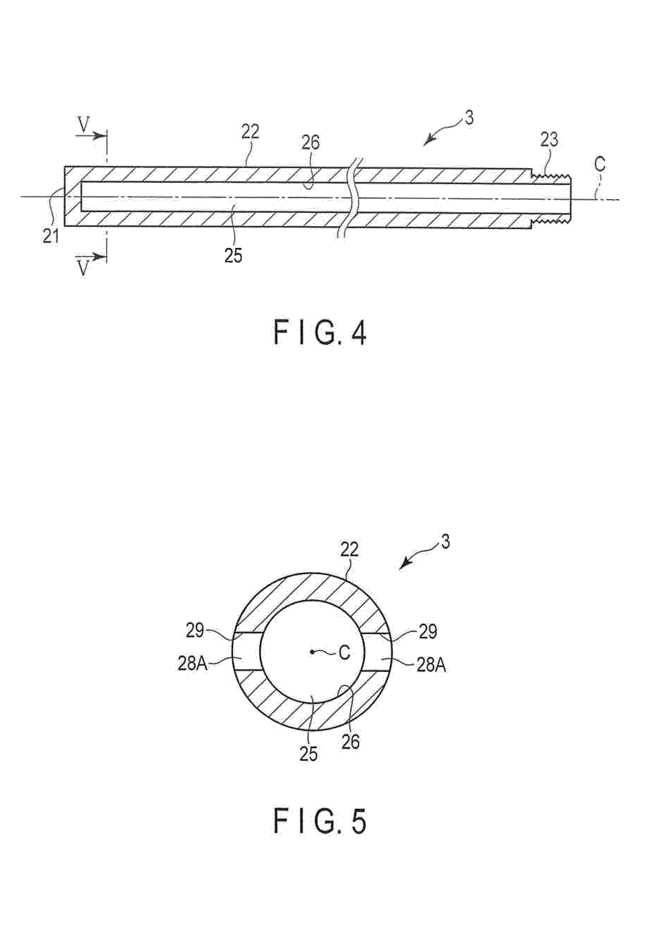

FIG. 3 and FIG. 4 are views showing a constitution of the probe 3. As shown in FIG. 3 and FIG. 4, the probe 3 is extended along the longitudinal axis C. The probe 3 includes a probe distal surface 21 and a probe outer peripheral portion 22. A distal end of the probe outer peripheral portion 22 forms an outer edge of the probe distal surface 21. A proximal end portion of the probe outer peripheral portion 22 is provided with an external thread portion 23 to be screwed into the internal thread portion 16 of the horn 15. When the external thread portion 23 is screwed into the internal thread portion 16, the probe 3 is attached to a distal direction side of the horn 15. When the probe 3 is attached to the horn 15, the ultrasonic vibration generated by ultrasonic vibrator 12 is transmitted to the probe distal surface 21 through the horn 15 and the probe 3. When the ultrasonic vibration is transmitted to the probe distal surface 21 and a liquid is supplied (forwarded) as described later, the living tissue is shattered (crushed) and emulsified with the probe distal surface 21 by utilizing a cavitation phenomenon. By the cavitation phenomenon, a tissue such as a blood vessel having a high elasticity is not shattered, and a living tissue of, for example, hepatic cells which do not have elasticity is shattered and emulsified.

It is to be noted that a length of an assembly of the probe 3, the ultrasonic vibrator 12 and the horn 15 along the longitudinal axis C is set so that the probe distal surface 21 of the probe 3 is an anti-node position of the ultrasonic vibration, and so that a proximal end of the ultrasonic vibrator 12 becomes an anti-node position of the ultrasonic vibration. When the probe distal surface 21 becomes the anti-node position of the ultrasonic vibration, the cavitation more efficiently takes place. Moreover, the ultrasonic vibration is longitudinal vibration in which a vibration transmitting direction is in parallel with a vibrating direction, and the vibration transmitting direction and the vibrating direction are parallel to the longitudinal axis C.

As shown in FIG. 4, in the probe 3, a suction passage 25 is defined by a suction passage defining portion 26. The suction passage 25 is extended along the longitudinal axis C from the distal end portion of the probe 3. A distal end of the suction passage 25 is positioned to a proximal direction side of the probe distal surface 21.

FIG. 5 is a cross-sectional view taken along line V-V of FIG. 4. As shown in FIG. 3 and FIG. 5, in the probe outer peripheral portion 22 of the probe 3, two suction openings (suction ports) 28A and 28B which communicate with the suction passage 25 are defined by a suction opening defining portion (a suction port defining portion) 29. Suction ports 28A and 28B are positioned apart from each other in directions around the longitudinal axis C. Moreover, a position of suction port 28A in directions parallel to the longitudinal axis C substantially coincides with (matches) a position of suction port 28B in the directions parallel to the longitudinal axis C. Furthermore, suction ports 28A and 28B are arranged in a vicinity of the probe distal surface 21.

When the probe 3 is attached to the horn 15, a proximal end of the suction passage 25 communicates with the passage portion 19 inside the ultrasonic vibrator 12 and the horn 15. As shown in FIG. 2, one end of a suction tube 31 is connected to the passage portion 19. As shown in FIG. 1, the suction tube 31 is extended to an outside from the vibrator case 11, and has the other end connected to the suction unit 33. The suction unit 33 is connected to the input unit 9. When the living tissue resected by the cavitation is suctioned, the suction unit 33 is driven by input in the input unit 9, or the like. When the suction unit 33 is driven, the resected living tissue is suctioned into the suction passage 25 through suction port 28A or suction port 28B. Then, the living tissue passes through the suction passage 25, the passage portion 19 and an inside of the suction tube 31 in this order, whereby the suction of the living tissue is accomplished by the suction unit 33.

As shown in FIG. 3 and FIG. 5, the probe distal surface 21 of the probe 3 is provided continuously from the longitudinal axis C to the outer edge thereof. That is, the probe distal surface 21 is not provided with any suction ports (distal suction ports) that communicate with the suction passage 25. Consequently, a surface area of the probe distal surface 21 increases. In this ultrasonic suction, the probe distal surface 21 is an action surface configured to shatter the living tissue by utilizing the cavitation phenomenon. Consequently, when the surface area of the probe distal surface 21 increases, an effective area where the cavitation phenomenon can be utilized increases. Therefore, the living tissue is efficiently shattered and emulsified.

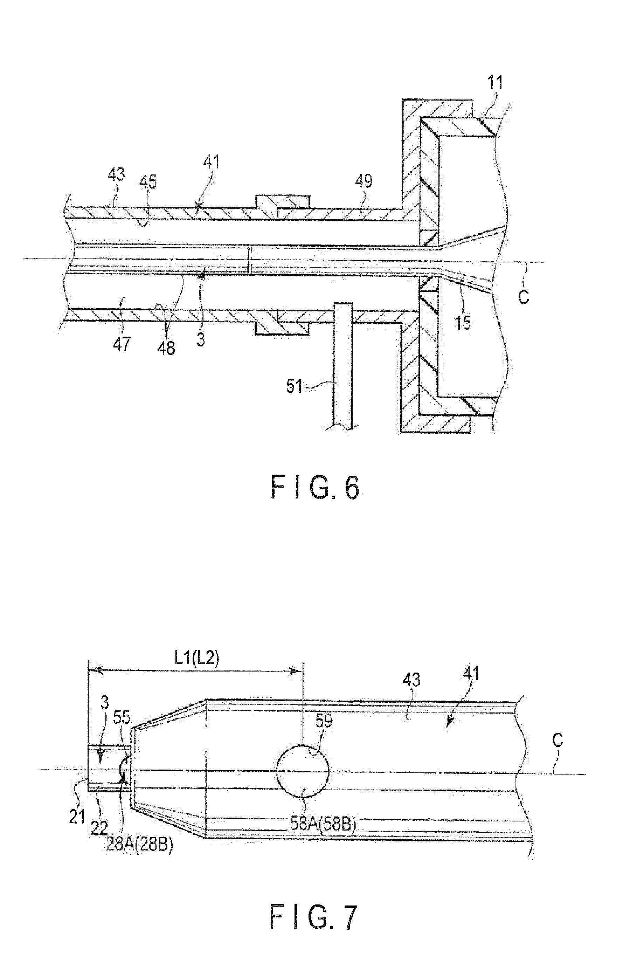

As shown in FIG. 1, the sheath unit 5 includes a sheath 41, and a holding case 42 configured to be held (grasped) by an operator. The sheath 41 is extended along the longitudinal axis C, and includes a sheath outer peripheral portion 43 and a sheath inner peripheral portion 45 as shown in FIG. 6. Moreover, the probe 3 is inserted through the sheath 41. The probe 3 is inserted through the sheath 41 so that the probe distal surface 21 is positioned to the distal direction side of a distal end of the sheath 41. In other words, the probe distal surface 21 (the distal end portion) of the probe 3 which is inserted through the sheath 41 is not covered with the sheath 41, but is exposed to the outside. The sheath 41 is inserted into the holding case 42 from the distal direction side, and the vibrator unit 2 is inserted into the holding case from the proximal direction side. In the holding case 42, the sheath 41 is connected to the vibrator case 11.

FIG. 6 is a view showing an inner constitution of the sheath 41 and a coupling constitution of the sheath 41 to the vibrator case 11. As shown in FIG. 6, between the sheath inner peripheral portion 45 of the sheath 41 and the probe outer peripheral portion 22 of the probe 3, a clearance 47 is defined by a clearance defining portion 48. Between the sheath inner peripheral portion 45 and the probe outer peripheral portion 22, the clearance 47 is extended along the longitudinal axis C from the distal end of the sheath 41. A distal end portion of a cylindrical intermediate member 49 is attached to a proximal end portion of the sheath 41. A distal end portion of the vibrator case 11 is attached to a proximal end portion of the intermediate member 49. It is to be noted that the sheath 41 may detachably be attached to the intermediate member 49 by, for example, screw fastening.

The clearance 47 between the sheath inner peripheral portion 45 and the probe outer peripheral portion 22 is extended up to the distal surface of the vibrator case 11. One end of a liquid supplying tube (liquid forwarding tube) 51 is connected to the intermediate member 49. An inside of the liquid supplying tube 51 communicates with the clearance 47. As shown in FIG. 1, the liquid supplying tube 51 is extended to the outside from the holding case 42, and is connected to the liquid supplying unit (liquid forwarding unit) 53. The liquid supplying unit 53 is connected to the input unit 9. When the liquid supplying unit 53 is driven by the input through the input unit 9 or the like, a liquid such as physiological saline passes through the inside of the liquid supplying tube 51 and the clearance 47 in this order. Then, the liquid is supplied (forwarded) to the living tissue or the like through a distal end of the clearance 47 (between the distal end of the sheath inner peripheral portion 45 and the probe outer peripheral portion 22).

As described above, in the ultrasonic suction, the ultrasonic vibration is transmitted to the probe distal surface 21 which is an anti-node position of the ultrasonic vibration. In this case, the liquid is supplied through the clearance 47, thereby causing the cavitation phenomenon. It is to be noted that the liquid may be supplied in a treatment other than the ultrasonic suction. For example, by supplying the liquid, confirmation of a bleeding spot, washing of a body cavity or the like may be performed.

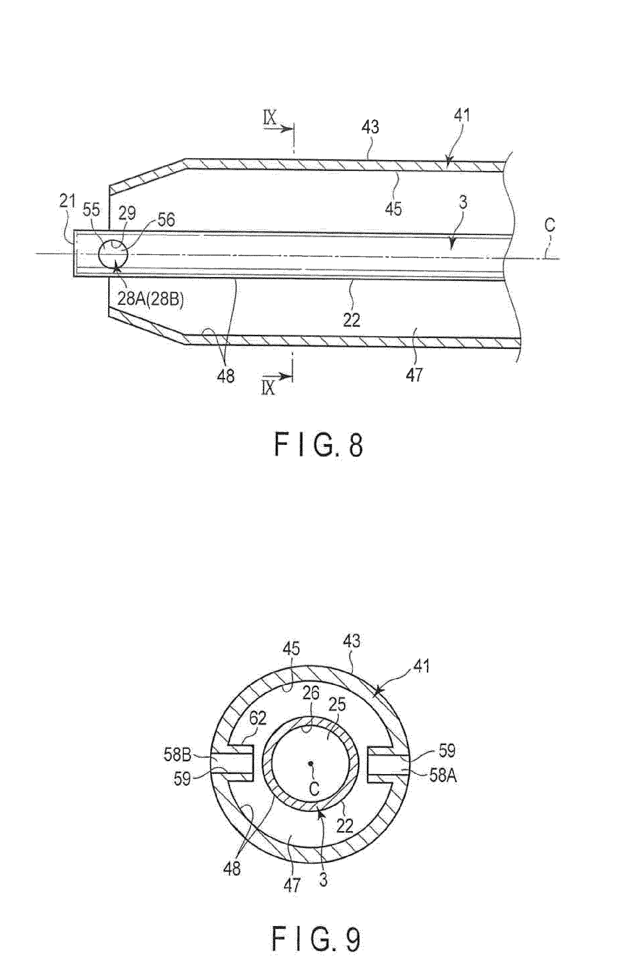

FIG. 7 and FIG. 8 show a constitution of the distal end portion of the probe 3 and the distal end portion of the sheath 41. FIG. 9 is a cross-sectional view taken along line IX-IX of FIG. 8. As shown in FIG. 7 and FIG. 8, suction port 28A of the probe 3 is provided at a position to face the distal end of the sheath 41. Moreover, suction port 28A of the probe 3 has an exposed portion 55 exposed to the outside, and a non-exposed portion 56 whose outer peripheral direction side is covered with the sheath 41. That is, the exposed portion 55 does not face the sheath inner peripheral portion 45 of the sheath 41, and the non-exposed portion 56 faces the sheath inner peripheral portion 45 of the sheath 41. In this way, a part of suction port 28A is the non-exposed portion 56 whose outer peripheral direction side is covered with the sheath 41. Similarly, a part of suction port 28B is the non-exposed portion 56 whose outer peripheral direction side is covered with the sheath 41.

As shown in FIG. 7 to FIG. 9, in the sheath outer peripheral portion 43 of the sheath 41, two openings 58A and 58B which communicate with the clearance 47 are defined by an opening defining portion 59. Openings 58A and 58B are positioned apart from each other in the directions around the longitudinal axis C. Moreover, a position of opening 58A in the directions parallel to the longitudinal axis C substantially coincides with (matches) a position of opening 58B in the directions parallel to the longitudinal axis C.

Each of dimensions L1 and L2 along the longitudinal axis C between each of openings 58A and 58B and the probe tip surface 21 is 2 cm or more and 10 cm or less. Moreover, a sum of areas of respective openings 58A and 58B is not less than a sum of areas of the non-exposed portions 56 of respective suction ports 28A and 28B. Furthermore, suction ports 28A and 28B are positioned to the distal direction side of the openings 58A and 58B.

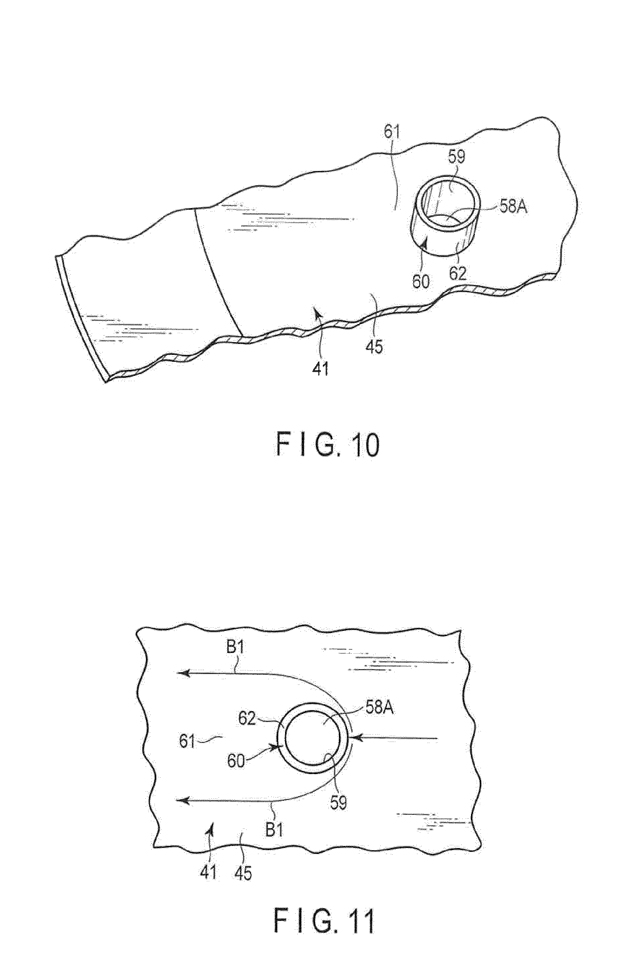

FIG. 10 and FIG. 11 are views showing a constitution of the sheath inner peripheral portion 45 in a vicinity of the one orifice 58A. As shown in FIG. 10 and FIG. 11, the sheath inner peripheral portion 45 includes a surface portion 61, and a convex portion 62 projecting toward (in) an inner peripheral direction from the surface portion 61. The surface portion 61 is a part of the sheath inner peripheral portion 45, and is formed into a curved or flat surface shape. The convex portion 62 is provided to surround a whole circumference of opening 58A, and an outer edge of opening 58A is formed by the convex portion 62.

As described above, in the case of the ultrasonic suction, the liquid (the physiological saline) from the liquid supplying unit 53 is supplied from the proximal direction to the distal direction through the clearance 47. In this case, the liquid collides with the convex portion 62 at a position to the proximal direction side of the opening 58A. Consequently, the liquid is guided so that the liquid passes a position away from the opening 58A (arrow B1 in FIG. 11). Therefore, inflow of liquid to the opening 58A is prevented. That is, an inflow prevention portion 60 configured to prevent inflow of liquid to the opening 58A is formed by the surface portion 61 and the convex portion 62. Similarly, also for the opening 58B, the inflow prevention portion 60 configured to prevent inflow of liquid to the opening 58B is provided.

Next, an action (function) of the ultrasonic treatment device 1 of the present embodiment will be described. When the ultrasonic suction of the living tissue is performed by using the ultrasonic treatment device 1, the current is supplied from the ultrasonic control section 8 to the ultrasonic vibrator 12 through the electrical signal lines 13A and 13B by an operation of the input unit 9, or the like. Consequently, the ultrasonic vibration takes place in the ultrasonic vibrator 12. Then, the ultrasonic vibration is transmitted to the probe distal surface 21 of the probe 3. Moreover, a liquid such as the physiological saline is supplied to the living tissue through the clearance 47 between the probe outer peripheral portion 22 and the sheath inner peripheral portion 45 by the liquid supplying unit 53. When the ultrasonic vibration is transmitted to the probe distal surface 21 and the liquid is supplied, the cavitation takes place. By the cavitation, the living tissue of, for example, the hepatic cells having low elasticity is selectively shattered (disintegrated) and resected.

Here, the probe distal surface 21 of the probe 3 is provided continuously from the longitudinal axis C to the outer edge thereof. Consequently, the surface area of the probe distal surface 21 increases. In the ultrasonic suction, the probe distal surface 21 is the action surface configured to shatter the living tissue by utilizing the cavitation phenomenon. Consequently, when the surface area of the probe distal surface 21 increases, the effective area where the cavitation phenomenon can be utilized increases. Therefore, the living tissue is efficiently shattered and emulsified.

Moreover, the liquid passing through the clearance 47 collides with the convex portion 62 at the position to the proximal direction side of the opening 58A. Consequently, the liquid is guided to pass the position away from the opening 58A (arrow B1 in FIG. 11). Therefore, inflow of liquid to the opening 58A is prevented. Similarly, also for the opening 58B, inflow of liquid to the opening 58B is prevented. As described above, outflow of the liquid to the outside of the sheath 41 through the openings 58A and 58B is effectively prevented. Therefore, the supplying of the liquid to the living tissue to be ultrasonically suctioned is efficiently performed.

Furthermore, the living tissue resected by the cavitation is suctioned. When the suction unit 33 is driven, the resected living tissue is suctioned into the suction passage 25 through suction port 28A or suction port 28B. Then, the living tissue passes through the suction passage 25, the passage portion 19 and the inside of the suction tube 31 in this order, whereby the suction of the living tissue is accomplished by the suction unit 33.

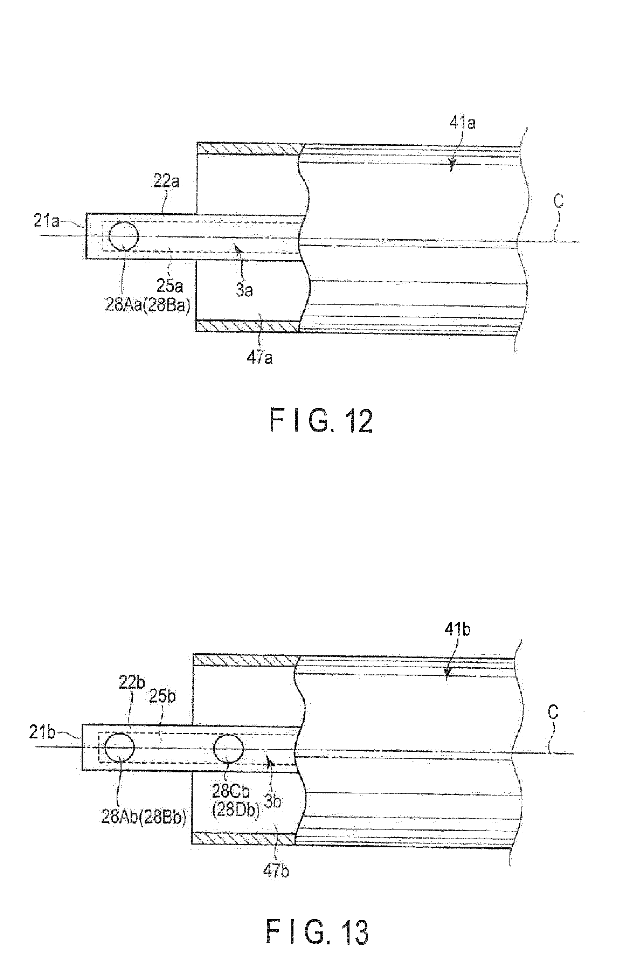

Here, as a first comparative example, a probe 3a and a sheath 41a shown in FIG. 12 are considered. In the first comparative example, respective suction ports 28Aa and 28Ba provided in the probe 3a are entirely exposed to the outside. That is, differently from suction ports 28A and 28B of the first embodiment, respective suction ports 28Aa and 28Ba are not provided with any non-exposed portions (56) whose outer peripheral direction sides are covered with the sheath 41a. Moreover, differently from the first embodiment, the sheath 41a is not provided with any openings (58A, 58B).

In the first comparative example, since respective whole suction ports 28Aa and 28Ba are exposed to the outside, the whole suction ports 28Aa and 28Ba are easily closed with a living tissue such as especially a membranous tissue in ultrasonic suction. Respective whole suction ports 28Aa and 28Ba are closed, whereby a suction passage 25a has a negative pressure lower than an external pressure. When the suction passage 25a is in the negative pressure state, the living tissue (the membranous tissue) firmly adheres to the probe 3a in the vicinities of a probe distal surface 21 and suction ports 28Aa and 28Ba of a probe outer peripheral portion 22a. As a result of the living tissue adhering to the probe 3a, treatment performance in terms of ultrasonic suction deteriorates.

Moreover, as a second comparative example, a probe 3b and a sheath 41b shown in FIG. 13 are considered. In the second comparative example, the probe 3b is provided with four suction ports 28Ab to 28Db. Respective whole suction ports 28Ab and 28Bb are exposed to the outside. The suction ports 28Cb and 28Db are positioned to a proximal direction side of the suction ports 28Ab and 28Bb. Outer peripheral direction sides of respective whole suction ports 28Cb and 28Db are covered with the sheath 41b. That is, respective whole suction ports 28Cb and 28Db are non-exposed portions (56). Moreover, differently from the first embodiment, the sheath 41b is not provided with any openings (58A, 58B).

In the second comparative example, respective whole suction ports 28Ab and 28Bb are exposed to the outside, and hence in the ultrasonic suction, respective whole suction ports 28Ab and 28Bb are easily closed with a living tissue such as a membranous tissue. However, the outer peripheral direction sides of respective whole suction ports 28Cb and 28Db are covered with the sheath 41b. Consequently, in the ultrasonic suction, suction ports 28Cb and 28Db are not closed with the living tissue. Therefore, a gas flows into a suction passage 25b from a clearance 47b between the probe 3b and the sheath 41b through suction port 28Cb or suction port 28Db.

However, in the second comparative example, the sheath 41b is not provided with the openings (58A, 58B). Therefore, when the gas flows into the suction passage 25b through the clearance 47b, the clearance 47b has a negative pressure lower than an external pressure. When the clearance 47b is in the negative pressure state, the living tissue (the membranous tissue) firmly adheres to the probe 3b and the sheath 41b in a vicinity of a distal end of the clearance 47b. As a result of the living tissue adhering to the probe 3b and the sheath 41b, treatment performance in terms of ultrasonic suction deteriorates.

To solve the problem, in the present embodiment, each of the suction ports 28A and 28B includes (has) the exposed portion 55 which is not covered with the sheath 41, and the non-exposed portion 56 which is covered with the sheath 41. In other words, the sheath 41 is set to such a length (dimension) that the distal end portion (the probe distal surface 21) of the probe 3 is exposed and that a part of each of suction ports 28A and 28B is covered, when the probe 3 is inserted through the sheath. Therefore, in the ultrasonic suction, the non-exposed portions 56 of suction ports 28A and 28B are not closed with the living tissue. Moreover, the sheath 41 is provided with the openings 58A and 58B which communicate with the clearance 47. Consequently, in the ultrasonic suction, the gas flows into the clearance 47 between the probe outer peripheral portion 22 and the sheath inner peripheral portion 45 from the outside of the sheath 41 through opening 58A or opening 58B. Then, the gas flows into the suction passage 25 of the probe 3 from the clearance 47 through the non-exposed portion 56 of suction port 28A or the non-exposed portion 56 of suction port 28B. Therefore, in the ultrasonic suction, the pressure of the clearance 47 and the suction passage 25 is about the same as the external pressure, and the clearance 47 and the suction passage 25 are not in the negative pressure state. Consequently, the adhesion of the living tissue to the probe 3 and the sheath 41 is effectively prevented, and the ultrasonic suction is efficiently performed.

Furthermore, the sum of the areas of respective openings 58A and 58B is not less than the sum of the areas of the non-exposed portions 56 of respective suction ports 28A and 28B. Consequently, in the ultrasonic suction, the amount of gas flowing into the clearance 47 from the outside of the sheath 41 through the opening 58A or the opening 58B becomes greater than the amount of gas flowing into the suction passage 25 from the clearance 47 through the non-exposed portion 56 of suction port 28A or the non-exposed portion 56 of suction port 28B. Therefore, change of the clearance 47 to the negative pressure state is further effectively prevented, and the adhesion of the living tissue to the probe 3 and the sheath 41 is further effectively prevented.

Moreover, suction ports 28A and 28B are positioned to the distal direction side of orifices 58A and 58B. That is, the suction ports 28A and 28B are positioned in the vicinity of the probe distal surface 21 of the probe 3. Consequently, the living tissue shattered and emulsified with the probe distal surface 21 by utilizing the cavitation is easily suctioned through suction port 28A or suction port 28B. Therefore, in the ultrasonic suction, the living tissue is efficiently suctioned.

Additionally, each of dimensions L1 and L2 along the longitudinal axis C between each of openings 58A and 58B and the probe distal surface 21 is 2 cm or more and 10 cm or less. When each of dimensions L1 and L2 is smaller than 2 cm, the positions of the openings (58A, 58B) come close to the living tissue to be treated by the ultrasonic suction. Consequently, the openings (58A, 58B) are easily closed with the living tissue (membranous tissue). When the openings (58A, 58B) are closed, the gas does not easily flow into the clearance 47 through the opening (58A or 58B), and the clearance 47 easily becomes the negative pressure state. Therefore, when each of dimensions L1 and L2 is 2 cm or more, the closing of openings 58A and 58B with the living tissue is effectively prevented. Consequently, the change of the clearance 47 to the negative pressure state is further effectively prevented.

On the other hand, when each of dimensions L1 and L2 is greater than 10 cm, the openings (58A, 58B) are positioned on an outside of a body in a treatment such as the ultrasonic suction. Consequently, at the treatment, the gas might flow into a body cavity from the outside of the body through the opening (58A or 58B). As a result of the gas flowing into the body cavity from outside the body, treatment performance may deteriorate. Therefore, each of dimensions L1 and L2 is set to 10 cm or less, whereby at the treatment, openings 58A and 58B are securely positioned in the body cavity. Consequently, during the treatment, inflow of gas from outside the body to the body cavity is effectively prevented, and deterioration of treatment performance is effectively prevented.

Accordingly, the ultrasonic treatment device 1 of the above constitution produces the following effects. That is, in the ultrasonic treatment device 1 of the present embodiment, a part of each of the suction ports 28A and 28B is the non-exposed portion 56 whose outer peripheral direction side is covered with the sheath 41. Consequently, in the ultrasonic suction, the non-exposed portions 56 of respective suction ports 28A and 28B are not closed with the living tissue. Moreover, the sheath 41 is provided with openings 58A and 58B which communicate with the clearance 47. Consequently, in the ultrasonic suction, the gas flows into the clearance 47 between the probe outer peripheral portion 22 and the sheath inner peripheral portion 45 from the outside of the sheath 41 through opening 58A or opening 58B. Then, the gas flows into the suction passage 25 of the probe 3 from the clearance 47 through the non-exposed portion 56 of suction port 28A or the non-exposed portion 56 of suction port 28B. Therefore, in the ultrasonic suction, the pressure of the clearance 47 and the suction passage 25 is about the same as the external pressure, and the clearance 47 and the suction passage 25 do not become the negative pressure state. Therefore, the adhesion of the living tissue to the probe 3 and the sheath 41 can be effectively prevented, and the ultrasonic suction can be efficiently performed.

Moreover, in the ultrasonic treatment device 1, the sum of the areas of respective openings 58A and 58B is not less than the sum of the areas of the non-exposed portions 56 of respective suction ports 28A and 28B. Therefore, in the ultrasonic suction, the amount of gas flowing into the clearance 47 from the outside of the sheath 41 through opening 58A or opening 58B is greater than the amount of gas flowing into the suction passage 25 from the clearance 47 through the non-exposed portion 56 of suction port 28A or the non-exposed portion 56 of suction port 28B. Therefore, the change of the clearance 47 to the negative pressure state can be further effectively prevented, and the adhesion of the living tissue to the probe 3 and the sheath 41 can be further effectively prevented.

Furthermore, in the ultrasonic treatment device 1, suction ports 28A and 28B are positioned to the distal direction side of openings 58A and 58B. That is, the suction ports 28A and 28B are positioned in the vicinity of the probe distal surface 21 of the probe 3. Consequently, the living tissue shattered and emulsified with the probe distal surface 21 by utilizing the cavitation is easily suctioned through suction port 28A or suction port 28B. Therefore, in the ultrasonic suction, the living tissue can be efficiently suctioned.

Additionally, in the ultrasonic treatment device 1, each of dimensions L1 and L2 along the longitudinal axis C between each of openings 58A and 58B and the probe distal surface 21 is 2 cm or more and 10 cm or less. When each of dimensions L1 and L2 is 2 cm or more, the closing of openings 58A and 58B with the living tissue can be effectively prevented. Consequently, the change of the clearance 47 to the negative pressure state can be further effectively prevented. Moreover, when each of dimensions L1 and L2 is 10 cm or less, openings 58A and 58B are securely positioned in the body cavity at the treatment. Consequently, during the treatment, inflow of gas from outside of the body to the body cavity can be effectively prevented, and deterioration of treatment performance can be effectively prevented.

Moreover, in the ultrasonic treatment device 1, the probe distal surface 21 of the probe 3 is provided continuously from the longitudinal axis C to the outer edge thereof. Consequently, the surface area of the probe distal surface 21 increases. In the ultrasonic suction, the probe distal surface 21 is the action surface configured to shatter the living tissue by utilizing the cavitation phenomenon. Consequently, when the surface area of the probe distal surface 21 increases, the effective area where the cavitation phenomenon can be utilized increases. Therefore, the living tissue can be efficiently shattered and emulsified.

Furthermore, in the ultrasonic treatment device 1, the liquid passing through the clearance 47 between the probe outer peripheral portion 22 and the sheath inner peripheral portion 45 collides with the convex portion 62 at the position to the proximal direction side of opening 58A. Consequently, the liquid is guided to pass the position away from the opening 58A (arrow B1 in FIG. 11). Therefore, inflow of liquid to opening 58A is prevented. Similarly, also for the opening 58B, inflow of liquid to opening 58B is prevented. As described above, the outflow of the liquid through the openings 58A and 58B to the outside of the sheath 41 can be effectively prevented. Therefore, the supplying of the liquid to the living tissue to be ultrasonically suctioned can be efficiently performed.

### (Modification of First Embodiment)

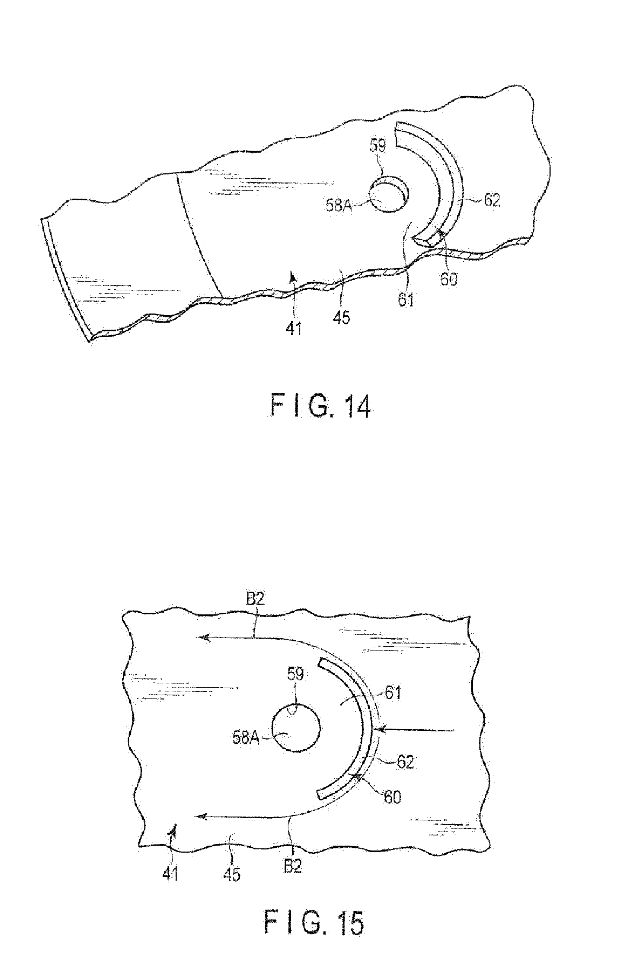

It is to be noted that in the first embodiment, the convex portion 62 is provided to surround the whole periphery of the opening 58A, and the outer edge of the opening 58A is formed by the convex portion 62, but the present invention is not limited to this embodiment. For example, as a first modification shown in FIG. 14 and FIG. 15, a convex portion 62 may be provided away from an opening 58A toward a proximal directione side. In the present modification, the whole periphery of the opening 58A is not surrounded by the convex portion 62. Moreover, the outer edge of opening 58A is not formed by the convex portion 62, and a surface portion 61 is provided (interposed) continuously between the opening 58A and the convex portion 62.

However, also in the present modification, similarly to the first embodiment, a liquid collides with the convex portion 62 at a position to the proximal direction side of opening 58A. Consequently, the liquid is guided to pass a position away from opening 58A (arrow B2 in FIG. 15). Therefore, inflow of liquid to the opening 58A is prevented. That is, an inflow prevention portion 60 configured to prevent inflow of liquid to the opening 58A is formed by the surface portion 61 and the convex portion 62. It is to be noted that also for an opening 58B, inflow of liquid to opening 58B may be prevented by using the inflow prevention portion 60 of the present modification.

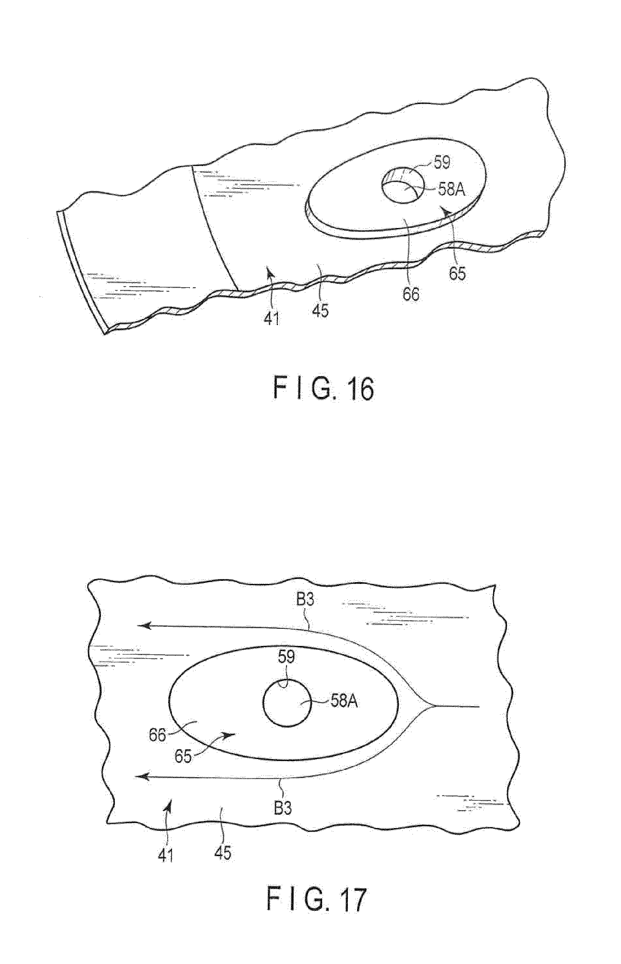

Moreover, in the first embodiment and the first modification, the inflow prevention portion 60 has the constitution including the surface portion 61 and the convex portion 62, but the present invention is not limited to these embodiments. For example, as a second modification shown in FIG. 16 and 17, inflow of liquid to an opening 58A may be prevented by an inflow prevention portion 65 having a constitution different from that of the inflow prevention portion 60. The inflow prevention portion 65 of the present modification includes a raised portion 66 which is provided in a sheath inner peripheral portion 45 with surrounding the opening 58A. The raised portion 66 is provided over a predetermined region around (about) opening 58A. In the raised portion, as it goes toward the opening 58A, the sheath inner peripheral portion 45 is positioned toward an inner peripheral direction side. When the raised portion 66 is provided, a liquid to be supplied through a clearance 47 is guided so that the liquid does not pass the raised portion 66 (arrow B3 in FIG. 17). That is, the liquid is guided to pass a position away from the opening 58A. Consequently, inflow of liquid to opening 58A is prevented. It is to be noted that also for an opening 58B, inflow of liquid to the opening 58B may be prevented by using the inflow prevention portion 65 of the present modification.

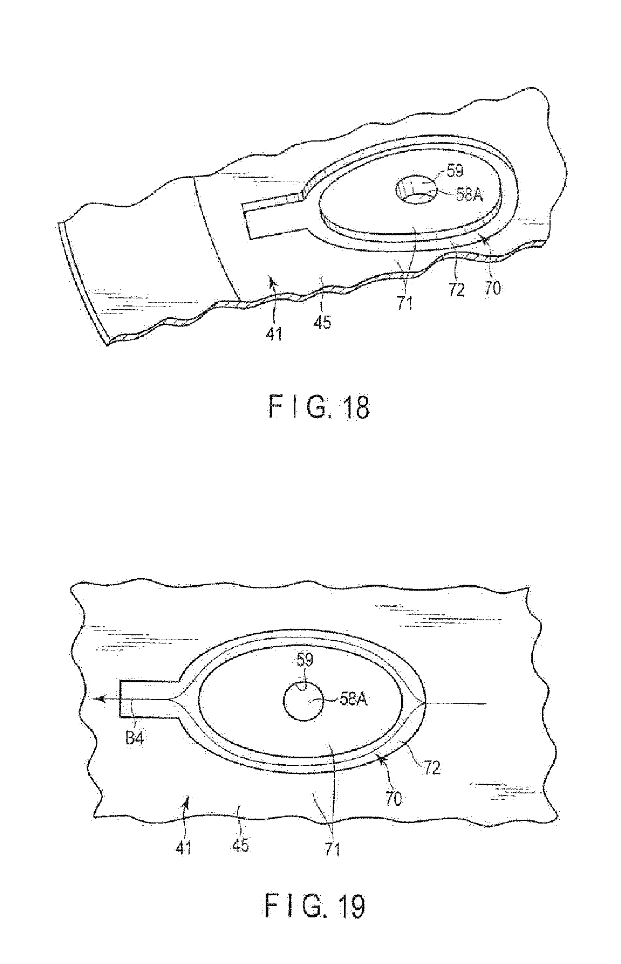

Furthermore, for example, as a third modification shown in FIG. 18 and FIG. 19, inflow of liquid to an opening 58A may be prevented by an inflow prevention portion 70 having a constitution different from those of the inflow prevention portions 60 and 65. The inflow prevention portion 70 of the present modification includes a surface portion 71, and a concave portion 72 in a dented state toward an outer peripheral direction side from the surface portion 71. The surface portion 71 becomes a part of a sheath inner peripheral portion 45, and is formed into a curved or flat surface shape. The concave portion 72 is provided at a position away from opening 58A. The concave portion 72 is extended from a position to a proximal direction side of the opening 58A up to a position to a distal direction side of the opening 58A. That is, a proximal end of the concave portion 72 is positioned to the proximal direction side of the opening 58A, and the distal end of the concave portion 72 is positioned to the distal direction side of the opening 58A.

A liquid to be supplied through a clearance 47 flows into the concave portion 72 at the position to the proximal direction side of the opening 58A. Then, the liquid flows along the concave portion 72, and is guided to the distal direction side of the opening 58A (arrow B4 in FIG. 19). Here, the concave portion 72 is provided at the position away from opening 58A. Consequently, the liquid which has flowed into the concave portion 72 is guided to pass the position away from opening 58A, by the concave portion 72. Consequently, inflow of liquid to the opening 58A is prevented. It is to be noted that also for an opening 58B, inflow of liquid to the opening 58B may be prevented by using the inflow prevention portion 70 of the present modification.

As understood from the above first to third modifications, the sheath inner peripheral portion 45 may be provided with the inflow prevention portion (60, 65, 70) configured to prevent inflow, to the opening (58A, 58B), of the liquid to be supplied from the proximal direction to the distal direction through the clearance 47.

Moreover, in the first embodiment, the two suction ports 28A and 28B are provided, and each of respective suction ports 28A and 28B is constituted of the exposed portion 55 and the non-exposed portion 56.

Furthermore, in the first embodiment, the two openings 58A and 58B are provided, but the present invention is not limited to this embodiment. Additionally, each of a shape of the suction port (28A, 28B) and a shape of the orifice (58A, 58B) is not limited to a circular shape which is the shape of the first embodiment.

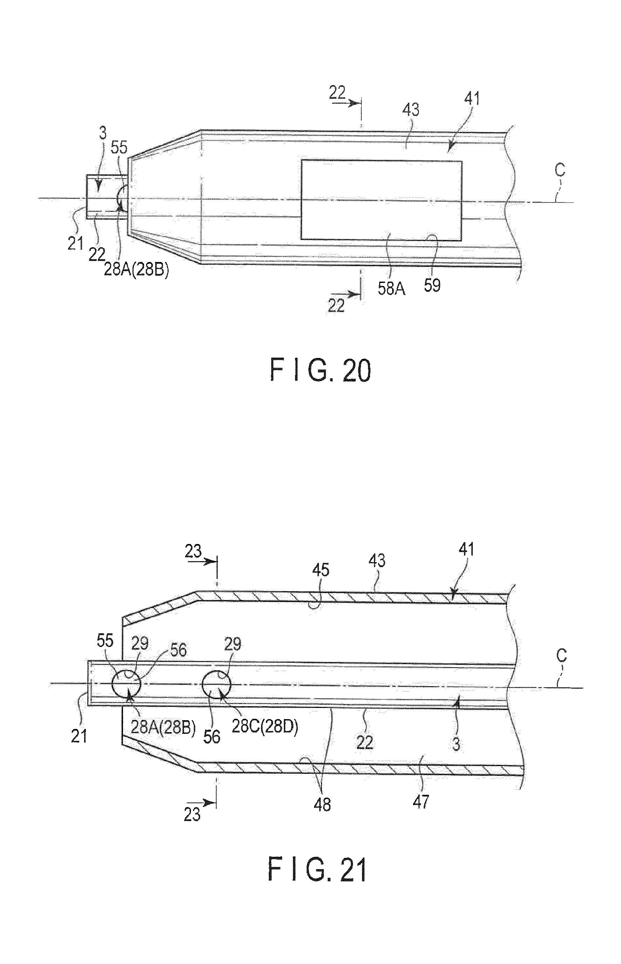

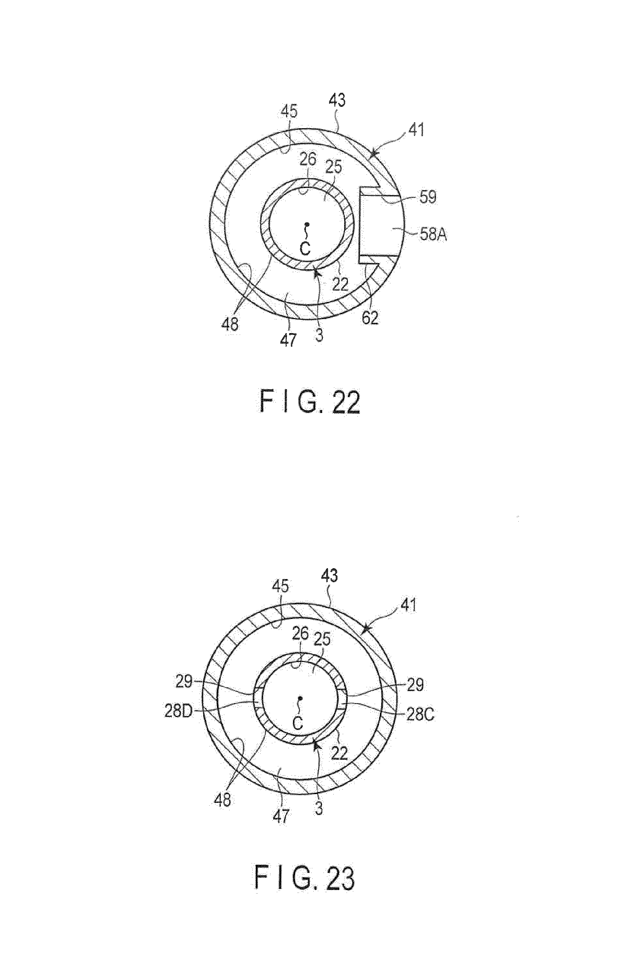

For example, as a fourth modification shown in FIG. 20 to FIG. 23, two suction ports 28C and 28D may further be provided at positions to a proximal direction side of suction ports 28A and 28B of a probe outer peripheral portion 22. Suction ports 28C and 28D are arranged apart from each other in directions around a longitudinal axis C. Moreover, a position of suction port 28C in directions parallel to the longitudinal axis C substantially coincides with a position of suction port 28D in the directions parallel to the longitudinal axis C. Each of respective suction ports 28A and 28B is constituted of an exposed portion 55 and a non-exposed portion 56 similarly to the first embodiment. Furthermore, each of respective suction ports 28C and 28D is constituted only of the non-exposed portion 56 whose outer peripheral direction side is covered with a sheath 41. That is, respective whole suction ports 28C and 28D are the non-exposed portions 56.

In a sheath outer peripheral portion 43, only one opening 58A having a substantially quadrangular shape is provided. Also in the present modification, the dimension along the longitudinal axis C between the opening 58A and a probe distal surface 21 is 2 cm or more and 10 cm or less. Moreover, also in the present modification, the area of the opening 58A is greater than the total area of the non-exposed portions 56 of respective suction port 28A to 28D. Furthermore, the suction ports 28A and 28B are positioned to a distal direction side of the opening 58A.

As understood from the above fourth modification, at least one suction port (28A-28D) may be provided to a probe outer peripheral portion 22. Moreover, at least a part (portion) of each of the suction port (28A-28D) may be the non-exposed portion 56 whose outer peripheral direction side is covered with the sheath 41. Furthermore, the sheath outer peripheral portion may be provided with at least one opening (58A, 58B). According to such a constitution, in ultrasonic suction, the pressure of a clearance 47 and a suction passage 25 is about the same as an external pressure, and the clearance 47 and the suction passage 25 do not attain a negative pressure state.

Moreover, the sum of the areas of the respective openings (58A, 58B) may be not less than the sum of the areas of the non-exposed portions 56 of the respective suction ports (28A-28D). Consequently, change of the clearance 47 to the negative pressure state is further effectively prevented. Furthermore, the suction ports (28A, 28B) positioned on the most distal direction side may be positioned to the distal direction side of the openings (58A, 58B) positioned on the most distal direction side. Consequently, a living tissue shattered and emulsified with the probe distal surface 21 by utilizing cavitation is easily suctioned through suction port 28A or suction port 28B. Furthermore, the dimension along the longitudinal axis C between each opening (58A, 58B) and the probe distal surface 21 may be 2 cm or more and 10 cm or less. Consequently, closing of openings 58A and 58B with the living tissue is effectively prevented, and at a treatment, inflow of gas from outside of the body to the body cavity is effectively prevented.

### (Illustrative Arrangement)

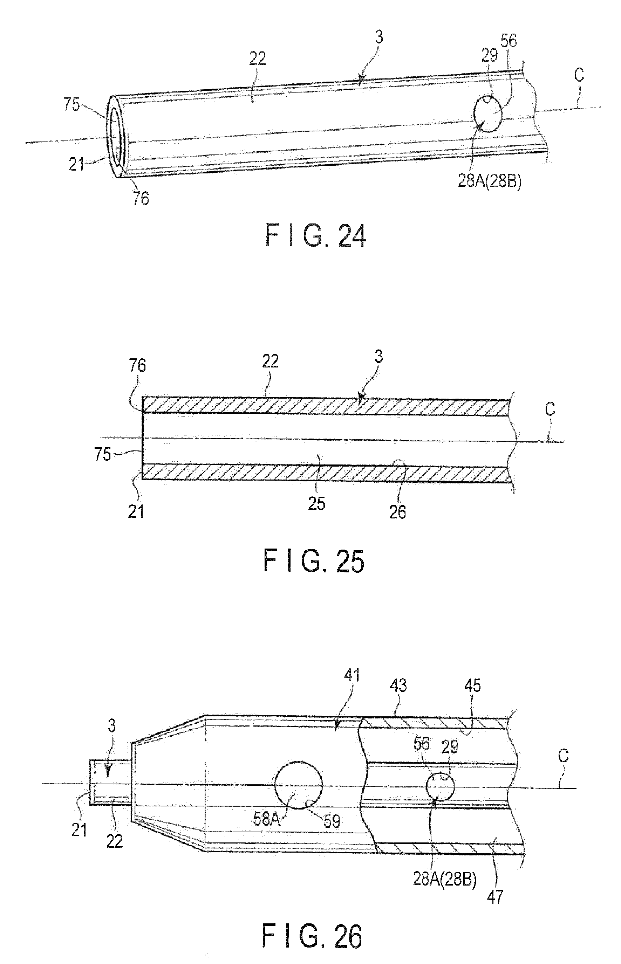

Next, an arrangement not part of the present invention will be described with reference to FIG. 24 to FIG. 26. In the arrangement, the constitution of the first embodiment is modified as follows. It is to be noted that a constitution common to the first embodiment is denoted with the same reference marks, and description of the constitution is omitted.

As shown in FIG. 24 and FIG. 25, according to an ultrasonic treatment device 1 of the present arrangement, in a probe distal surface 21, a distal suction port 75 which communicates with a suction passage 25 is defined by a distal suction port defining portion 76. That is, the probe distal surface 21 is provided discontinuously from a longitudinal axis C to an outer edge thereof.

As shown in FIG. 26, in the present arrangement, suction ports 28A and 28B are positioned to a proximal direction side of openings 58A and 58B. Each of respective suction ports 28A and 28B is constituted only of a non-exposed portion 56 whose outer peripheral direction side is covered. That is, respective whole suction ports 28A and 28B are non-exposed portions 56.

It is to be noted that also in the present arrangement, similarly to the first embodiment, a sum of areas of respective openings 58A and 58B is not less than a sum of areas of non-exposed portions 56 of respective suction ports 28A and 28B. Moreover, a dimension along the longitudinal axis C between each of respective openings 58A and 58B and the probe distal surface 21 is 2 cm or more and 10 cm or less.

In the present arrangement the probe distal surface 21 is provided with the distal suction port 75. Consequently, it is not necessary to suction, through suction port 28A or suction port 28B, a living tissue shattered and emulsified with the probe distal surface 21 by utilizing cavitation, and the living tissue is easily suctioned through the distal suction port 75. Therefore, in ultrasonic suction, the living tissue can be efficiently suctioned through the distal suction port 75 irrespective of the positions of suction ports 28A and 28B.

Incidentally, the present invention is not limited to the above embodiments and various modifications can naturally be made within the scope of independent claim 1.

## Claims

1. An ultrasonic treatment device (1) comprising:
a sheath (41) which includes a sheath outer peripheral portion (43) and a sheath inner peripheral portion (45), and which is extended along a longitudinal axis (C);
a probe (3) which includes a probe distal surface (21) and a probe outer peripheral portion (22), and which is inserted through the sheath (41) in a state that the probe distal surface (21) is positioned to a distal direction (A1) side of a distal end of the sheath (41);
a suction passage defining portion (26) which defines a suction passage (25) along the longitudinal axis (C) inside the probe (3); and
a clearance defining portion (48) which defines a clearance (47) between the sheath inner peripheral portion (45) and the probe outer peripheral portion (22);
a suction port defining portion (29) which defines at least one suction port (28A, 28B) in the probe outer peripheral portion (22), each suction port (28A, 28B) communicating with the suction passage and
an opening defining portion (59) which defines at least one opening (58A, 58B; 58A) in the sheath outer peripheral portion (43), each opening (58A, 58B; 58A) communicating with the clearance (47), **characterised in that**;
each suction port (28A, 28B) has a non-exposed portion (56) whose outer peripheral direction side is covered with the sheath (41), and an exposed portion (55) whose outer peripheral direction side is uncovered by the sheath (41); and **in that** a gas being configured to flow into the clearance (47) from an outside of the sheath (41) through each of the at least one opening (58A, 58B; 58A) and then the gas flowed into the clearance (47) being configured to flow into the suction passage (25) through the non-exposed portion (56) of each suction port (28A, 28B) when the suction force is caused in the suction passage (25).

2. The ultrasonic treatment device (1) according to claim 1,
**characterized in that**
the opening defining portion (59) defines the at least one opening (58A, 58B; 58A) so that a sum of an area of a respective opening (58A, 58B; 58A) is not less than a sum of an area of the non-exposed portion (56) of a respective suction port (28A, 28B).

3. The ultrasonic treatment device (1) according to claim 1,
**characterized in that** the sheath inner peripheral portion (45) includes an inflow prevention portion (60; 65; 70) configured to prevent inflow, to the opening (58A, 58B; 58A) of a liquid to be supplied from a proximal direction (A2) to the distal direction (A1) through the clearance (47).

4. The ultrasonic treatment device (1) according to claim 3,
**characterized in that** the inflow prevention portion (60) includes:
a surface portion (61) which is a part of the sheath inner peripheral portion (45); and
a convex portion (62) which projects toward an inner peripheral direction from the surface portion (61), and with which the liquid is configured to collide at a position located to a proximal direction (A2) side of the at least one opening (58A, 58B; 58A), the convex portion (62) being configured to guide the collided liquid so that the liquid passes a position away from the at least one opening (58A, 58B; 58A).

5. The ultrasonic treatment device (1) according to claim 3,
**characterized in that** the inflow prevention portion (65) includes a raised portion (66) which is provided in the sheath inner peripheral portion (45) and which surrounds the at least one opening (58A, 58B; 58A), a position in the raised portion (66) being a more inner peripheral position as the position in the raised portion (66) is nearer the at least one opening (58A, 58B; 58A), and the raised portion (66) being configured to guide the liquid so that the liquid passes a position away from the opening (58A, 58B; 58A).

6. The ultrasonic treatment device (1) according to claim 3,
**characterized in that** the inflow prevention portion (70) includes:
a surface portion (71) which becomes a part of the sheath inner peripheral portion (45); and
a concave portion (72) which is provided at a position away from the at least one opening (58A, 58B; 58A) and which concaves from the surface portion (71), the liquid being configured to inflow into the concave portion (72) at a position located to the proximal direction (A2) side of the at least one opening (58A, 58B; 58A), the concave portion (72) being configured to flow the inflowed liquid along the concave portion (72) and thereby configured to guide the inflowed liquid so that the liquid passes the position away from the at least one opening (58A, 58B; 58A).

7. The ultrasonic treatment device (1) according to claim 1,
**characterized in that** the probe distal surface (21) is provided continuously from the longitudinal axis (C) to an outer edge thereof,
and the at least one suction port (28A, 28B) is positioned distally to the at least one opening (58A,58B; 58A).

## Patentansprüche

1. Ultraschallbehandlungsgerät (1), das umfasst:
eine Hülse (41), die einen Außenumfangshülsenabschnitt (43) und einen Innenumfangshülsenabschnitt (45) umfasst und die sich entlang einer Längsachse (C) erstreckt;
eine Sonde (3), die eine distale Sondenoberfläche (21) und einen Außenumfangssondenabschnitt (22) umfasst und die durch die Hülse (41) in einem Zustand eingeführt ist, dass die distale Sondenoberfläche (21) zu einer Seite einer distalen Richtung (A1) eines distalen Endes der Hülse (41) positioniert ist;
einen Saugkanaldefinitionsabschnitt (26), der einen Saugkanal (25) entlang der Längsachse (C) innerhalb der Sonde (3) definiert; und
einen Spaltdefinitionsabschnitt (48), der einen Spalt (47) zwischen dem Innenumfangshülsenabschnitt (45) und dem Außenumfangssondenabschnitt (22) definiert;
einen Sauganschlussdefinitionsabschnitt (29), der mindestens einen Sauganschluss (28A, 28B) in dem Außenumfangssondenabschnitt (22) definiert, wobei jeder Sauganschluss (28A, 28B) mit dem Saugkanal kommuniziert, und
einen Öffnungsdefinitionsabschnitt (59), der mindestens eine Öffnung (58A, 58B; 58A) in dem Außenumfangshülsenabschnitt (43) definiert, wobei jede Öffnung (58A, 58B; 58A) mit dem Spalt (47) kommuniziert,
**dadurch gekennzeichnet, dass**
jeder Sauganschluss (28A, 28B) einen nicht-exponierten Abschnitt (56), dessen Außenumfangsrichtungsseite von der Hülse (41) abgedeckt ist, und einen exponierten Abschnitt (55), dessen Außenumfangsrichtungsseite nicht von der Hülse (41) abgedeckt ist, umfasst; und dadurch, dass
ein Gas, das von außerhalb der Hülse (41) durch jede der mindestens einen Öffnung (58A, 58B; 58A) in den Spalt (47) zu strömen vermag und das in den Spalt (47) geströmte Gas dann durch den nicht-exponierten Abschnitt (56) eines jeden Sauganschlusses (28A, 28B) zu strömen vermag, wenn die Saugkraft in dem Saugkanal (25) verursacht wird.

2. Ultraschallbehandlungsgerät (1) gemäß Anspruch 1,
**dadurch gekennzeichnet, dass**
der Öffnungsdefinitionsabschnitt (59) die mindestens eine Öffnung (58A, 58B; 58A) derart definiert, dass eine Summe einer Fläche einer jeweiligen Öffnung (58A, 58B; 58A) nicht kleiner ist als eine Summe einer Fläche des nicht-exponierten Abschnitts (56) eines jeweiligen Sauganschlusses (28A, 28B).

3. Ultraschallbehandlungsgerät (1) gemäß Anspruch 1,
**dadurch gekennzeichnet, dass** der Innenumfangshülsenabschnitt (45) einen Einströmungsverhinderungsabschnitt (60; 65; 70) umfasst, der dazu eingerichtet ist, ein Einströmen einer Flüssigkeit zu der Öffnung (58A, 58B; 58A) zu verhindern, die dazu vorgesehen ist, von einer proximalen Richtung (A2) zu der distalen Richtung (A1) durch den Spalt (47) zugeführt zu werden.

4. Ultraschallbehandlungsgerät (1) gemäß Anspruch 3,
**dadurch gekennzeichnet, dass** der Einströmungsverhinderungsabschnitt (60) umfasst:
einen Oberflächenabschnitt (61), der ein Teil des Innenumfangshülsenabschnitts (45) ist; und
einen konvexen Abschnitt (62), der in einer Innenumfangsrichtung von dem Oberflächenabschnitt (61) vorsteht und mit dem die Flüssigkeit an einer Position zu kollidieren vermag, die zu einer Seite einer proximalen Richtung (A2) der mindestens einen Öffnung (58A, 58B; 58A) angeordnet ist, wobei der konvexe Abschnitt (62) dazu eingerichtet ist, die kollidierte Flüssigkeit so zu führen, dass die Flüssigkeit an einer von der mindestens einen Öffnung (58A, 58B; 58A) entfernten Position vorbei geleitet wird.

5. Ultraschallbehandlungsgerät (1) gemäß Anspruch 3,
**dadurch gekennzeichnet, dass** der Einströmungsverhinderungsabschnitt (65) einen erhöhten Abschnitt (66) umfasst, der in dem Innenumfangshülsenabschnitt (45) vorgesehen ist und der die mindestens eine Öffnung (58A, 58B; 58A) umgibt, wobei eine Position in dem erhöhten Abschnitt (66) eine weiter innen am Umfang gelegene Position ist, wenn die Position in dem erhöhten Abschnitt (66) näher an der mindestens einen Öffnung (58A, 58B; 58A) liegt und der erhöhte Abschnitt (66) dazu eingerichtet ist, die Flüssigkeit so zu führen, dass die Flüssigkeit an einer von der mindestens einen Öffnung (58A, 58B; 58A) entfernten Position vorbei geleitet wird.

6. Ultraschallbehandlungsgerät (1) gemäß Anspruch 3,
**dadurch gekennzeichnet, dass** der Einströmungsverhinderungsabschnitt (70) umfasst:
einen Oberflächenabschnitt (71), der ein Teil des Innenumfangshülsenabschnitts (45) wird; und
einen konkaven Abschnitt (72), der an einer von der mindestens einen Öffnung (58A, 58B; 58A) entfernten Position vorgesehen ist und der von dem Oberflächenabschnitt (71) konkav geformt ist, wobei die Flüssigkeit an einer Position, die zu einer Seite einer proximalen Richtung (A2) der mindestens einen Öffnung (58A, 58B; 58A) angeordnet ist, in den konkaven Abschnitt (72) einzuströmen vermag, wobei der konkave Abschnitt (72) dazu eingerichtet ist, die eingeströmte Flüssigkeit entlang des konkaven Abschnitts (72) strömen zu lassen und dadurch dazu eingerichtet ist, die eingeströmte Flüssigkeit so zu führen, dass die Flüssigkeit an einer von der mindestens einen Öffnung (58A, 58B; 58A) entfernten Position vorbei geleitet wird.

7. Ultraschallbehandlungsgerät (1) gemäß Anspruch 1,
**dadurch gekennzeichnet, dass** die distale Sondenoberfläche (21) kontinuierlich von der Längsachse (C) zu deren Außenrand vorgesehen ist und der mindestens eine Sauganschluss (28A, 28B) distal zu der mindestens einen Öffnung (58A, 58B; 58A) positioniert ist.

## Revendications

1. Dispositif de traitement par ultrasons (1) comprenant :
une gaine (41) qui comprend une partie périphérique externe de gaine (43) et une partie périphérique interne de gaine (45), et qui s'étend le long d'un axe longitudinal (C) ;
une sonde (3) qui comprend une surface distale de sonde (21) et une partie périphérique externe de sonde (22), et qui est insérée à travers la gaine (41) dans un état dans lequel la surface digitale de sonde (21) est positionnée vers un côté de direction distale (A1) d'une extrémité distale de la gaine (41) ;
une partie définissant un passage d'aspiration (26) qui définit un passage d'aspiration (25) le long de l'axe longitudinal (C) à l'intérieur de la sonde (3) ; et
une partie définissant un jeu (48) qui définit un jeu (47) entre la partie périphérique interne de gaine (45) et la partie périphérique externe de sonde (22) ;
une partie définissant un orifice d'aspiration (29) qui définit au moins un orifice d'aspiration (28A, 28B) dans la partie périphérique externe de sonde (22), chaque orifice d'aspiration (28A, 28B) communiquant avec le passage d'aspiration, et
une partie définissant une ouverture (59) qui définit au moins une ouverture (58A, 58B ; 58A) dans la partie périphérique externe de gaine (43), chaque ouverture (58A, 58B ; 58A) communiquant avec le jeu (47),
**caractérisé en ce que**
chaque orifice d'aspiration (28A, 28B) comporte une partie non exposée (56) dont le côté de direction périphérique externe est recouvert par la gaine (41), et une partie exposée (55) dont le côté de direction périphérique externe n'est pas recouvert par la gaine (41) ; et **en ce que**
un gaz étant configuré pour circuler dans le jeu (47) depuis l'extérieur de la gaine (41) à travers chacune parmi la au moins une ouverture (58A, 58B ; 58A) et ensuite le gaz ayant circulé dans le jeu (47) est configuré pour circuler dans le passage d'aspiration (25) à travers la partie non exposée (56) de chaque orifice d'aspiration (28A, 28B) lorsque la force d'aspiration est provoquée dans le passage d'aspiration (25).

2. Dispositif de traitement par ultrasons (1) selon la revendication 1,
**caractérisé en ce que**
la partie définissant une ouverture (59) définit la au moins une ouverture (58A, 58B ; 58A) de sorte qu'une somme d'une zone d'une ouverture (58A, 58B ; 58A) respective n'est pas inférieure à une somme d'une zone de la partie non exposée (56) d'un orifice d'aspiration (28A, 28B) respectif.

3. Dispositif de traitement par ultrasons (1) selon la revendication 1,
**caractérisé en ce que** la partie périphérique interne de gaine (45) comprend une partie empêchant un débit entrant (60 ; 65 ; 70) configurée pour empêcher un débit entrant, vers l'ouverture (58A, 58B ; 58A) d'un liquide devant être délivré depuis une direction proximale (A2) vers la direction distale (A1) par l'intermédiaire du jeu (47).

4. Dispositif de traitement par ultrasons (1) selon la revendication 3,
**caractérisé en ce que** la partie empêchant un débit entrant (60) comprend :
une partie de surface (61) qui est une partie de la partie périphérique interne de gaine (45) ; et
une partie convexe (62) qui fait saillie vers une direction périphérique interne depuis la partie de surface (61), et avec laquelle le liquide est configuré pour entrer en collision au niveau d'une position placée vers un côté de direction proximale (A2) de la au moins une ouverture (58A, 58B ; 58A), la partie convexe (62) étant configurée pour guider le liquide s'étant heurté de sorte que le liquide passe par une position éloignée de la au moins une ouverture (58A, 58B ; 58A).

5. Dispositif de traitement par ultrasons (1) selon la revendication 3,
**caractérisé en ce que** la partie empêchant un débit entrant (65) comprend une partie élevée (66) qui est prévue dans la partie périphérique interne de gaine (45) et qui entoure la au moins une ouverture (58A, 58B ; 58A), une position de la partie élevée (66) étant une position périphérique plus interne étant donné que la position de la partie élevée (66) est plus proche de la au moins une ouverture (58A, 58B ; 58A), et la partie élevée (66) étant configurée pour guider le liquide de sorte que le liquide passe par une position éloignée de l'ouverture (58A, 58B ; 58A).

6. Dispositif de traitement par ultrasons (1) selon la revendication 3,
**caractérisé en ce que** la partie empêchant un débit entrant (70) comprend :
une partie de surface (71) qui devient une partie de la partie périphérique interne de gaine (45) ; et
une partie concave (72) qui est prévue au niveau d'une position éloignée de la au moins une ouverture (58A, 58B ; 58A) et qui est concave par rapport à la partie de surface (71), le liquide étant configuré pour entrer dans la partie concave (72) au niveau d'une position placée vers le côté de direction proximale (A2) de la au moins une ouverture (58A, 58B ; 58A), la partie concave (72) étant configurée pour faire circuler le liquide étant entré le long de la partie concave (72) et de ce fait configurée pour guider le liquide étant entré de sorte que le liquide passe par la position éloignée de la au moins une ouverture (58A, 58B ; 58A).

7. Dispositif de traitement par ultrasons (1) selon la revendication 1,
**caractérisé en ce que** la surface distale de sonde (21) est prévue de manière continue de l'axe longitudinal (C) à un bord externe de celle-ci,
et le au moins un orifice d'aspiration (28A, 28B) est positionné de manière distale par rapport à la au moins une ouverture (58A, 58B ; 58A).
